# FASCICULE DE BREVET EUROPEEN

(11) **EP 1 971 268 B1**
(45) Date de publication et mention de la délivrance du brevet: **23.12.2009**
(21) Numéro de dépôt: 07717689.9
(22) Date de dépôt: 05.01.2007
(51) Int. Cl.: A61B 10/00

(54) **DISPOSITIF DE DETERMINATION DE L'ETAT PHYSIOLOGIQUE LIE A LA PROCREATION CHEZ LES MAMMIFERES FEMELLES**
VORRICHTUNG ZUR BESTIMMUNG DES PHYSIOLOGISCHEN ZUSTANDES WEIBLICHER SÄUGER IN ZUSAMMENHANG MIT FORTPFLANZUNG
DEVICE FOR DETERMINING THE PHYSIOLOGICAL STATE OF FEMALE MAMMALS RELATED TO PROCREATION

(30) Priorité: 05.01.2006 FR 0600151
(43) Date de publication de la demande: 24.09.2008
(73) Titulaire: La société "EFFIANCE", 43100 Brioude (FR)
(72) Inventeur: VINCI, René, F-66000 Perpignan (FR)
(74) Mandataire: Rhein, Alain
(86) Numéro de dépôt international: PCT/FR2007/050615
(87) Numéro de publication internationale: WO 2007/077405

(56) Documents cités:
- WO-A-94/23653
- WO-A-02/087446
- DE-A1- 19 943 456
- US-A- 6 080 118

## Description

L'invention concerne un dispositif de détermination de l'état physiologique lié à la procréation chez les bovins femelles.

L'invention entre dans le domaine de la détermination de l'état physiologique lié à la procréation chez les bovins femelles.

L'état physiologique lié à la procréation chez les bovins femelles comprend notamment des états particuliers significatifs, comme l'état fécond, la gestation ou encore l'imminence de la mise bas.

Chez les bovins femelles en général, l'état fécond correspond à la période des chaleurs.

La gestation intervient dès qu'une fécondation a eu lieu et s'achève par la mise bas.

Il est très utile de pouvoir déterminer avec exactitude la survenue de tels états, et leurs intervalles de durée. Pour l'état fécond, ceci est intéressant notamment dans l'optique d'une insémination artificielle pour obtenir une fécondation maîtrisée. En effet, les échecs de fécondation artificielle restent nombreux et la présence ou non d'un état fécond en est une des variables. Ces échecs ont en outre un coût important.

Physiologiquement, l'entrée dans une période où l'ovule est fécondable ou sur le point de l'être se traduit par des variations de température et de concentrations en hormones dans le vagin des bovins femelles.

De la même manière, l'étude de ces paramètres physiologiques et de leurs variations peut permettre de détecter une gestation, faisant par exemple suite à une insémination réussie, ou encore l'imminence de la mise bas qui sont tout aussi intéressants à prévoir.

On connaît déjà des dispositifs pour évaluer notamment l'état fécond d'un bovin femelle. Les plus simples utilisent une sonde manuelle de mesure de la température génitale, d'autres mesurent les paramètres physiques de la muqueuse vaginale en introduisant manuellement une sonde de mesure.

Ces dispositifs ne sont tout d'abord pas d'usage commode et donnent des résultats discutables.

On connaît également des dispositifs de détermination de l'état physiologique lié à la procréation chez les mammifères femelles de conception plus complexe. Ainsi le document US 6 080 118 décrit une sonde utilisée pour déterminer différents états corporels d'un patient humain ou d'un animal, et qui comprend à cet effet un certain nombre de biocapteurs et/ou de capteurs de température.

Le document WO 94/23653 décrit un appareil électronique de surveillance de l'ovulation, comportant un boîtier contenant un dispositif de traitement d'informations, telles que la densité des muqueuses, la température basale, le pH et le taux de LH.

Le document DE 199 43 456 décrit un système télémétrique de surveillance de l'ovulation chez les femmes. Il comporte une ou plusieurs sondes et une unité de transmission. La ou les sondes sont disposées dans un pessaire flexible.

Le document WO 02/087446 concerne un procédé de détermination de l'état hormonal des femmes, en particulier, du moment de l'ovulation. Le procédé consiste à mesurer dans le vagin, en continu ou à des intervalles de temps réguliers déterminés, via la conductance, la concentration en ions de la muqueuse cervicale, le cas échéant, le pH de la sécrétion vaginale et la température vaginale.

Outre leur complexité, ces dispositifs ne sont pas utilisables de manière automatisée, notamment sur les grands élevages de bovins.

La présente invention se propose de répondre à ces inconvénients majeurs dans le domaine de la détermination de l'état physiologique lié à la procréation chez les bovins femelles.

La présente invention s'adresse aux bovins femelles en général.

A cet effet l'invention concerne un dispositif de détermination de l'état physiologique lié à la procréation chez les bovins femelles, comprenant un boîtier étanche destiné à être introduit dans l'appareil génital d'un bovin femelle, et comportant d'une part en sa périphérie et/ou intérieurement au moins deux capteurs de données physiques et/ou physiologiques différentes, et d'autre part des moyens d'alimentation électrique l'autorisant à fonctionner de manière autonome; et associé à des moyens de traitement des données recueillies par lesdits capteurs ainsi que des moyens aptes à diffuser les données collectées et/ou les résultats issus dudit traitement des données, et il se caractérise en ce que l'un desdits capteurs de données physiques et/ou physiologiques est constitué par un capteur de mouvement, tandis qu'un autre desdits capteurs de données physiques et/ou physiologiques est constitué par un capteur acoustique sensible aux mugissements en sorte de permettre une exploitation du changement de spectre desdits mugissements.

Avantageusement, l'un des capteurs de données physiques et/ou physiologiques du boîtier est constitué par un capteur de température.

L'un des capteurs de données physiques et/ou physiologiques peut encore être constitué par un capteur permettant de déterminer la présence et/ou la concentration en hormones.

Le boîtier peut en outre comprendre des moyens de diffusion d'une substance dans l'appareil génital du bovin femelle.

L'invention permet, grâce à l'étude simultanée d'au moins deux paramètres, notamment la température et la concentration en hormones dans le vagin d'un bovin femelle, de déterminer de manière précise un des états physiologiques lié à la procréation, tels un état d'ovulation et la durée de la période de fécondité et/ou un état de gestation et/ou l'imminence de la mise bas.

En outre, elle permet avantageusement d'exploiter automatiquement et rapidement des données physiques, physiologiques et biologiques prélevées in situ dans l'appareil génital d'un bovin femelle.

D'autres caractéristiques, buts et avantages de la présente invention ressortiront de la description détaillée qui suit, en référence aux dessins annexés, se rapportant à deux exemples de réalisation donnés à titre indicatif et non limitatif, dans lesquels :
- la figure 1 présente un mode particulier de réalisation du dispositif de détection de l'état fécond selon l'invention ;
- la figure 2 présente une variante du mode de réalisation de la figure 1;
- la figure 3 présente un autre mode de réalisation du dispositif de détection de l'état fécond selon l'invention.

On notera que dans la description qui suit certains modes de réalisation ne sont pas revendiqués, et sont seulement décrites à titre informatif.

Tel que visible sur les dessins annexés, la présente invention concerne le domaine de la détermination de l'état physiologique lié à la procréation chez les bovins femelles, et a trait, plus particulièrement, à un dispositif de détermination de l'état physiologique lié à la procréation chez les bovins femelles.

Plus précisément, l'invention vise à permettre la détection de paramètres visant la détermination notamment l'état fécond et/ou la gestation et/ou l'imminence de la mise bas chez les bovins femelles.

Grâce à une étude temporelle de paramètres physiologiques accessibles du vagin, il est possible de déterminer le moment où l'ovule est fécondable ou sur le point de l'être, ce qui se traduit notamment par des variations de température et de concentrations en hormones dans le vagin.

De manière similaire, il est possible de détecter une gestation ou l'imminence d'une mise bas.

C'est ainsi particulièrement l'étude de ces variations qui permet de déterminer une entrée dans un état fécond, une gestation ou l'imminence de la mise bas.

L'invention est destinée à permettre de détecter les valeurs de certains paramètres physiologiques, notamment afin de les étudier dans le temps.

Pour plus de clarté, l'exposé détaillé suivant se réfèrera principalement à la détection de l'état fécond des bovins femelles. La transposition à la détection d'une gestation ou de l'imminence d'une mise bas est aisée, la seule différence se trouvant dans l'analyse, éventuellement dans le temps, des valeurs détectées et/ou le choix des paramètres à observer.

Les paramètres d'étude préférentiels sont constitués par la température, accessible directement, et la concentration en hormones, notamment en progestérone.

En effet et à ce propos, le processus d'ovulation s'accompagne d'une augmentation du taux de progestérone permettant de détecter une ovulation et donc un état fécond.

De manière générale, il est naturellement possible de détecter d'autres hormones dans le vagin de bovins femelles, pouvant servir la détermination de l'état physiologique lié à la procréation chez les bovins femelles.

L'étude de la concentration en hormones peut se faire directement. Dans le cas de la progestérone, elle peut également être obtenue par l'observation d'effets secondaires consécutifs aux variations de concentration en progestérone. Il s'agit par exemple de la mesure de la résistance ou résistivité de la muqueuse vaginale ou du mucus, qui est directement liée à la concentration en progestérone.

Plus particulièrement, ce sont les dates ou instants correspondant à des variations caractéristiques, comme une brusque élévation ou une brusque diminution, des paramètres mentionnés plus haut qui renseignent sur un état fécond.

En outre, l'invention tire parti d'une exploitation simultanée des variations d'au moins deux paramètres, notamment la température et la concentration en hormones, cette exploitation conduisant à déterminer un état d'ovulation ou son imminence.

Afin d'étudier les variations de ces paramètres, on utilise un dispositif 1 comprenant au moins un boîtier 2 étanche destiné à être introduit dans l'appareil génital d'un bovin femelle, ledit boîtier 2 comportant en sa périphérie et/ou intérieurement au moins deux capteurs différents 3. Le boîtier 2 inclut des moyens d'alimentation électrique 4 l'autorisant à fonctionner de manière autonome, en particulier sous forme d'une pile 4, et des moyens de traitement 5 des informations fournies par les capteurs 3. Naturellement, le dispositif 1 comprend encore des moyens aptes à diffuser les données collectées et/ou les résultats issus dudit traitement des données.

Ces derniers moyens aptes à diffuser les données collectées et/ou les résultats issus dudit traitement des données permettent la matérialisation des données collectées et/ou les résultats issus du traitement des données sur tout type de support, à l'aide par exemple d'un écran d'ordinateur, d'une imprimante ou encore sous forme de signaux lumineux ou sonores.

Préférentiellement, les deux types au moins de capteurs 3 employés permettent d'une part de mesurer la température, et d'autre part la concentration en hormones. Il est naturellement possible d'employer en plus des capteurs 3 aux fonctions différentes, ainsi que nous le verrons plus loin.

On aura également compris que, sans que l'invention y soit limitée, l'étude de la concentration en progestérone est particulièrement utile.

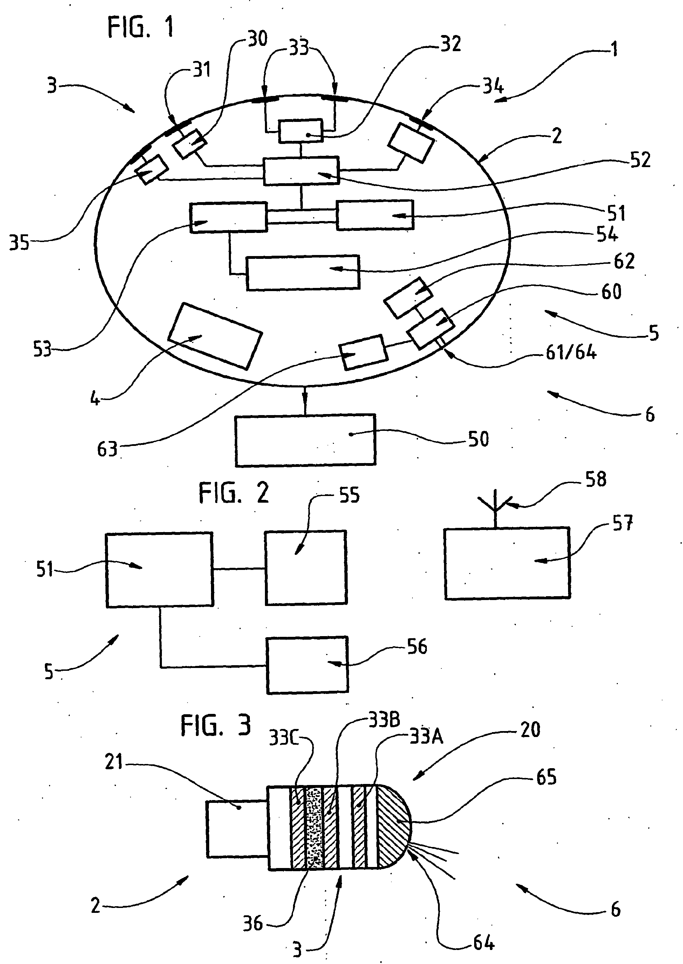

La figure 1 présente un mode de réalisation particulier du dispositif selon l'invention, comprenant un capteur de température 30 et sa sonde 31 à placer en contact avec la paroi vaginale. On pourra également imaginer un capteur de température entièrement isolé dans le boîtier 2.

Différents types de capteurs peuvent être envisagés, et ceci particulièrement pour la mesure de la concentration en hormones.

Ainsi, la variation de la concentration en hormones est commodément et préférentiellement accessible par la mesure de la résistance ou résistivité de la muqueuse et/ou du mucus.

Le mode de réalisation de la figure 1 présente un boîtier 2 incorporant un capteur 32 sensible à la résistance de la muqueuse vaginale avec au moins 2 électrodes 33 à placer en contact avec la paroi vaginale et/ou le mucus.

La figure 3, qui présente un mode de réalisation du boîtier 2 selon l'invention, comprend également un capteur 32 permettant de mesurer la résistance de la muqueuse vaginale entre les électrodes 33A et 33B et la résistance du revêtement ou mucus entre les électrodes 33B et 33C.

La variation de la concentration en hormones est encore accessible par des biocapteurs possédant des sites moléculaires permettant la fixation d'hormones, ladite fixation induisant une variation de grandeurs électriques.

Ainsi le mode de réalisation de la figure 1 présente encore un biocapteur 34 sensible aux estrogènes et un biocapteur 35 réagissant aux phéromones.

Comme biocapteur, on pourra employer un revêtement qui, s'imprégnant d'hormones, par exemple par absorption, voit sa résistivité changer.

Dans le mode de réalisation de la figure 3, le boîtier 2 comprend encore un capteur 36 sous forme d'un revêtement bioactif 36 capable de réagir avec les molécules organiques présentes dans le vagin et la muqueuse.

Ainsi qu'expliqué plus haut, ces deux résistances informent finalement, par leurs valeurs mesurées ou leurs variations, sur la présence et/ou l'évolution de la concentration en hormones, dans le mucus et le vagin.

Selon un autre avantage de l'invention et afin d'apporter une solution automatisée à la détection de l'état fécond chez les bovins femelles, des moyens de traitement 5 des informations fournies par les capteurs 3 ont été intégrés au dispositif 1 selon l'invention.

Ces moyens de traitement 5 se trouvent préférentiellement répartis entre l'intérieur et l'extérieur du boîtier 2.

Les mesures relevées par les capteurs 3 peuvent ainsi être directement traitées dans le boîtier 2 et/ou transmises par télémétrie à une unité centrale 50 extérieure. Le résultat du traitement des données vise naturellement la détermination de l'état fécond ou non du bovin femelle testé et/ou la détermination d'une période de fécondité.

De la même manière on pourra rechercher une gestation et/ou l'imminence d'une mise bas.

Le traitement direct des mesures à l'intérieur du boîtier 2 est effectué par le biais d'au moins une mémoire 51 reliée à une unité de contrôle 52, permettant de mémoriser, à intervalles réguliers, par exemple la température, la résistance de la paroi vaginale, la concentration en oestrogènes ou encore la présence de phéromones, ces paramètres dépendant des capteurs 3 présents.

Une unité centrale 53 située l'intérieur du boîtier 2 et comportant de la mémoire et un logiciel de traitement permet ensuite d'assurer l'analyse de ces mesures relevées par les capteurs 3 et le séquencement des opérations de mesure et de mémorisation.

Le logiciel de traitement permet notamment de déterminer avec précision une période de fécondité.

Avantageusement, une fois l'analyse de ces mesures faites, le résultat de celles-ci est transmis par télémétrie via une unité d'émission-réception 54 assurant le dialogue et le transfert des résultats avec l'unité centrale 50 fixe. L'unité d'émission-réception 54 comprend avantageusement un module émetteur-récepteur.

A ce propos, l'unité centrale 53 gère également le séquencement de l'émission vers l'unité centrale 50 extérieure.

Selon une variante du dispositif 1 selon l'invention, le transfert des mesures effectuées pourra également se faire sans analyse préalable à l'intérieur du boîtier 2, ou en complément du transfert de l'analyse effectuée à l'intérieur du boîtier 2.

Selon le mode particulier de réalisation de la figure 1, les valeurs mesurées sont relevées à des dates et horaires fixes et mémorisés dans la mémoire 51.

Ces valeurs sont ensuite soit exploitées localement par le logiciel de traitement de l'unité centrale 53, soit transmises à l'unité centrale 50 extérieure, par exemple, dans le cas d'une application à des troupeaux d'animaux, lors du passage de chaque animal devant un portique de réception où à des distances depuis le lieu du pâturage.

Comme vu précédemment, diverses options sont possibles, comme la transmission finale uniquement de l'analyse opérée par l'unité centrale 53, ou à la fois de cette analyse et des mesures relevées au niveau des capteurs 3, ou seulement les mesures relevées au niveau des capteurs 3, demandant éventuellement encore un traitement ultérieur et extérieur au boîtier 2 pour déterminer l'état de fécondité et/ou de gestation et/ou l'imminence d'une mise bas.

Dans ce contexte, le résultat de l'analyse, permettant de déterminer en particulier une période propice à la fécondation, est ensuite accessible aux responsables de la reproduction qui peuvent décider ou non de tenter une insémination.

Ainsi, le dispositif de la présente invention permet de connaître, avec une bonne probabilité, les périodes de fécondité des femelles d'un troupeau d'animaux bovins d'élevage, limitant au mieux les échecs et le coût de l'insémination artificielle, ou encore de tenir à l'écart les mâles dans ces mêmes périodes.

Ceci est également très utile pour préparer une éventuelle intervention vétérinaire dans le cas d'une mise bas.

La figure 2 montre une variante du dispositif 1 selon la figure 1, dans laquelle les données de la mémoire 51 sont écrites dans un module RFID 55 par un module d'écriture 56.

A l'extérieur du boîtier 2, un module 57 d'excitation et de lecture du RFID avec son antenne 58 permet de lire des données contenues dans le module RFID 55 lié à la mémoire 51 et de les traiter. Le module 57 permet encore d'écrire dans ledit module RFID 55.

Cette variante faisant appel à la radio-identification est particulièrement pertinente pour la surveillance de troupeaux d'animaux. L'exemple suivant se rapporte à un troupeau de bovins. Chaque vache est équipée à demeure d'un boîtier 2 qui émet en permanence à intervalles réguliers un code numérique qui lui est propre. Dès réception de ce code, l'unité centrale 50 fixe, disposée dans l'étable, envoie un signal au boîtier 2 lui demandant d'émettre l'historique, des valeurs des paramètres relevés sous forme de courbes. Cet historique est capté par l'unité centrale 50 et traitée pour mettre à jour une des informations « ovulation », « gestation » ou « imminence d'une mise bas » contenue dans la variation des paramètres relevés, et ceci de manière individuelle pour chaque vache.

Avantageusement, tel que représenté en figure 1, le boîtier 2 du dispositif selon l'invention comprend encore des moyens de diffusion 6 d'une substance dans l'appareil génital du bovin femelle sous forme d'au moins une électrovanne 60 capable d'injecter, par une buse 61, et sous contrôle d'une unité de commande 62, une substance contenue dans un réservoir 63 sous pression. Ladite unité de commande 62 peut, préférentiellement, se trouver sous contrôle distant, et ainsi être excitable de l'extérieur du boîtier 2.

L'unité de commande 62 peut encore se trouver sous contrôle de l'unité centrale 53.

Dans un cas où l'autre, elle peut encore être excitable de manière automatisée en fonction des valeurs, ou résultats analysés, résultants des mesures relevées par les capteurs 3.

La substance diffusée peut, mais non exclusivement, présenter une activité bactéricide.

En particulier, la substance diffusée peut être une molécule provoquant la mise bas.

Le mode de réalisation de la figure 3 présente également un tel boîtier 2 pourvu de tels moyens de diffusion 6, attesté par la présence d'au moins un orifice d'éjection 64.
L'homme du métier concevra sans mal un boîtier 2 pourvu de plusieurs jeux d'électrovannes avec leurs réservoirs afin, par exemple, de diffuser plusieurs substances différentes. A cet effet, le boîtier 2 comprendra avantageusement plusieurs orifices reliés aux électrovannes.

Le mode de réalisation du dispositif présenté en figure 3, qui comporte avantageusement un boîtier 2 en deux parties, l'une 20 comportant les capteurs 3 et l'autre 21 l'électronique de traitement des mesures effectuées, présente encore d'autres caractéristiques particulières.

En effet, les moyens de diffusion 6 d'une substance dans l'appareil génital du bovin femelle sous forme d'une électrovanne 60 sont complétés par un revêtement diffuseur et/ou bactéricide 65. Cette option pourra naturellement être préférée et choisie exclusivement par rapport à la présence d'un système de diffusion avec électrovanne, plus complexe.

Pour en revenir aux paramètres susceptibles d'être exploités, d'autres paramètres que la température ou la concentration en hormones pourront être relevés par le boîtier 2, moyennant des capteurs 3 adaptés. En venant en sus de la température ou la concentration d'hormones, ces paramètres peuvent ainsi permettre d'affiner la détection.

En fonction des besoins ou disponibilités, l'homme du métier pourra également sélectionner les paramètres à exploiter, afin d'assurer la fiabilité de l'analyse, qu'elle soit automatisée ou non.

En effet, il apparaîtra évident à l'homme du métier de moduler la composition en capteurs du dispositif en fonction des fonctionnalités desdits capteurs choisis.

D'autres paramètre pertinemment exploitables sont par exemple le rythme cardiaque ou, dans le cas de l'utilisation du dispositif chez des animaux, la fréquence du mouvement des pattes en déplacement, ou encore le changement du spectre des mugissements.

Dans cette optique, d'autres capteurs intérieurs au boîtier 2 sont ainsi nécessaires, tel un capteur acoustique non représenté, sensible en particulier au bruit des battements du coeur tant du bovin femelle ou à des mugissements.

Un capteur de pression affleurant, non représenté sur les figures permet également d'enregistrer le rythme cardiaque.

Un tel capteur permet également de détecter des contractions dans le cas d'une imminence d'une mise bas.

Un capteur de mouvement sensible aux déplacements pourra faire office de podomètre afin d'évaluer leur agitation.

Enfin, un capteur de type caméra pourra utilement servir à visualiser et quantifier la dilatation du col.

On rappelle que selon l'invention, les mêmes paramètres que ceux mesurés pour la détection d'un état fécond pourront avantageusement également permettre de détecter un état de gestation, faisant par exemple suite à une insémination artificielle réussie où encore l'imminence d'une mise bas.

Dans cette optique et selon une variante du dispositif selon l'invention, le boîtier 2 pourra encore comprendre un générateur d'ultrasons.

## Revendications

1. Dispositif (1) de détermination de l'état physiologique lié à la procréation chez les bovins femelles, comprenant un boîtier (2) étanche destiné à être introduit dans l'appareil génital d'un bovin femelle, et comportant d'une part en sa périphérie et/ou intérieurement au moins deux capteurs (3) de données physiques et/ou physiologiques différentes, et d'autre part des moyens d'alimentation électrique (4) l'autorisant à fonctionner de manière autonome; et associé à des moyens de traitement (5) des données recueillies par lesdits capteurs (3) ainsi que des moyens aptes à diffuser les données collectées et/ou les résultats issus dudit traitement des données, **caractérisé en ce que** l'un desdits capteurs (3) de données physiques et/ou physiologiques est constitué par un capteur de mouvement, tandis qu'un autre desdits capteurs (3) de données physiques et/ou physiologiques est constitué par un capteur acoustique sensible aux mugissements en sorte de permettre une exploitation du changement de spectre desdits mugissements.

2. Dispositif (1) selon la revendication 1, **caractérisé en ce que** l'un des capteurs (3) de données physiques et/ou physiologiques est constitué par un capteur de température (30).

3. Dispositif (1) selon l'une des revendications précédentes, **caractérisé en ce que** l'un des capteurs (3) de données physiques et/ou physiologiques est constitué par un capteur (3) permettant de déterminer la présence et/ou la concentration en hormones.

4. Dispositif (1) selon l'une des revendications précédentes, **caractérisé en ce que** l'un des capteurs (3) de données physiques et/ou physiologiques est constitué par un capteur (32) permettant de déterminer la résistance et/ou résistivité de la muqueuse vaginale et/ou le mucus.

5. Dispositif (1) selon l'une des revendications précédentes, **caractérisé en ce que** l'un des capteurs (3) de données physiques et/ou physiologiques est constitué par un capteur (34) sensible aux oestrogènes.

6. Dispositif (1) selon l'une des revendications précédentes, **caractérisé en ce que** l'un des capteurs (3) de données physiques et/ou physiologiques est constitué par un revêtement bioactif (36) sur le boîtier (2).

7. Dispositif (1) selon l'une des revendications précédentes, **caractérisé en ce que** l'un des capteurs (3) de données physiques et/ou physiologiques est constitué par un capteur de pression.

8. Dispositif (1) selon l'une des revendications précédentes, **caractérisé en ce que** l'un des capteurs (3) de données physiques et/ou physiologiques est constitué par un capteur de type caméra.

9. Dispositif (1) selon l'une des revendications précédentes, **caractérisé en ce que** les moyens de traitement (5) des données sont constitués par au moins une mémoire (51), au moins une unité de contrôle (52) de la mémoire, et au moins une unité centrale (53) assurant le traitement des données provenant du ou des capteurs (3) de données physiques et/ou physiologiques.

10. Dispositif (1) selon la revendication 9, **caractérisé en ce que** l'unité centrale (53) est située à l'intérieur du boîtier (2) et comporte de la mémoire et un logiciel de traitement.

11. Dispositif (1) selon l'une des revendications précédentes, **caractérisé en ce que** les moyens de traitement (5) des données comprennent une unité d'émission-réception (54) de signaux à l'intérieur du boîtier (2) et une unité centrale extérieure (50), lesdites unités (54,50) étant conçues aptes à dialoguer.

12. Dispositif (1) selon l'une des revendications précédentes, **caractérisé en ce que** le boîtier (2) inclut en plus des moyens de diffusion (6) d'une substance dans l'appareil génital du bovin femelle.

13. Dispositif selon la revendication 12, **caractérisé en ce que** les moyens de diffusion (6) d'une substance dans l'appareil génital du bovin femelle sont constitués par au moins une électrovanne (60) associée à un réservoir (63) sous pression.

14. Dispositif (1) selon la revendication 13, **caractérisé en ce que** l'électrovanne (60) associée au réservoir (63) sous pression est contrôlée par une unité de commande (62).

15. Dispositif (1) selon la revendication 12, **caractérisé en ce que** les moyens de diffusion (6) d'une substance dans l'appareil génital du bovin femelle sont constitués par au moins un revêtement diffuseur et/ou bactéricide (65) disposé à la périphérie du boîtier (2).

16. Dispositif (1) selon l'une des revendications précédentes, **caractérisé en ce que** le boîtier (2) comprend en plus un générateur d'ultrasons.

## Claims

1. Device (1) for determining the physiological state related to procreation in female bovine animals, comprising a sealed casing (2) aimed at being inserted into the reproductive system of a female bovine animal, and including, on the one hand, on its periphery and/or internally at least two different physical and/or physiological data sensors (3) and, on the other hand, current-supply means (4) enabling same to operate autonomously; and associated with means for processing (5) the data collected by said sensors (3) as well as means capable of spreading the collected data and/or the results proceeding from said data processing, wherein one of said physical and/or physiological data sensors (3) is formed of a movement sensor, while another one of said physical and/or physiological data sensors (3) is formed of a lowing-sensitive acoustic sensor so as to allow using the change in the spectrum of said lowing.

2. Device (1) according to claim 1, wherein one of the physical and/or physiological data sensors (3) is formed of a temperature sensor (30).

3. Device (1) according to one of the preceding claims, wherein one of the physical and/or physiological data sensors (3) is formed of a sensor (3) permitting to determine the presence and/or the concentration of hormones.

4. Device (1) according to one of the preceding claims, wherein one of the physical and/or physiological data sensors (3) is formed of a sensor (32) permitting to determine the resistance and/or the resistivity of the vaginal mucosa and/or the mucus.

5. Device (1) according to one of the preceding claims, wherein one of the physical and/or physiological data sensors (3) is formed of an oestrogen-sensitive sensor (34).

6. Device (1) according to one of the preceding claims, wherein one of the physical and/or physiological data sensors (3) is formed of a bioactive coating (36) on the casing (2).

7. Device (1) according to one of the preceding claims, wherein one of the physical and/or physiological data sensors (3) is formed of a pressure sensor.

8. Device (1) according to one of the preceding claims, wherein one of the physical and/or physiological data sensors (3) is formed of a camera-type sensor.

9. Device (1) according to one of the preceding claims, wherein the means for processing (5) the data are formed of at least one memory (51), at least one unit for controlling (52) the memory, and at least one central unit (53) ensuring the processing of the data proceeding from the physical and/or physiological data sensor or sensors (3).

10. Device (1) according to claim 9, wherein the central unit (53) is located inside the casing (2) and includes memory and processing software.

11. Device (1) according to one of the preceding claims, wherein the means for processing (5) the data comprise a signal transmitting-receiving unit (54) inside the casing (2) and an external central unit (50), said units (54, 50) being designed capable of dialoguing.

12. Device (1) according to one of the preceding claims, wherein the casing (2) includes in addition means for diffusing (6) a substance into the reproductive system of the female bovine animal.

13. Device (1) according to claim 12, wherein the means for diffusing (6) a substance into the reproductive system of the female bovine animal are formed of at least one solenoid valve (60) associated with a tank (63) under pressure.

14. Device (1) according to claim 13, wherein the solenoid valve (60) associated with the tank (63) under pressure is controlled by a control unit (62).

15. Device (1) according to claim 12, wherein the means for diffusing (6) a substance into the reproductive system of the female bovine animal are formed of at least one diffusing and/or bactericide coating (65) arranged on the periphery of the casing (2).

16. Device (1) according to one of the preceding claims, wherein the casing (2) comprises in addition an ultrasound generator.

## Patentansprüche

1. Vorrichtung (1) zum Bestimmen des physiologischen Zustandes weiblicher Rinder in Zusammenhang mit der Fortpflanzung, umfassend ein dichtes Gehäuse (2), das dazu bestimmt ist, in das Geschlechtssystem eines weiblichen Rindes eingeführt zu werden, und umfassend, einerseits, auf seinem Umkreis und/oder inwendig wenigstens zwei verschiedene physikalische und/oder physiologische Datensensoren (3) und, andererseits, Stromversorgungsmittel (4), die es diesen letzteren erlauben, selbständig zu arbeiten; und die Mitteln zum Verarbeiten (5) der von den besagten Sensoren (3) gesammelten Daten zugeordnet sind, sowie Mittel, die geeignet sind, die gesammelten Daten und/oder die von der besagten Datenverarbeitung stammenden Ergebnisse zu diffundieren, **dadurch gekennzeichnet, dass** einer der besagten physikalischen und/oder physiologischen Datensensoren (3) aus einem Bewegungssensor besteht, während ein anderer der besagten physikalischen und/oder physiologischen Datensensoren (3) aus einem gebrüllempfindlichen akustischen Sensor besteht, um die Verwendung der Spektrumänderung des besagten Gebrülls zu erlauben.

2. Vorrichtung (1) nach Anspruch 1, **dadurch gekennzeichnet, dass** einer der besagten physikalischen und/oder physiologischen Datensensoren (3) aus einem Temperatursensor (30) besteht.

3. Vorrichtung (1) nach einem der vorgehenden Ansprüche, **dadurch gekennzeichnet, dass** einer der besagten physikalischen und/oder physiologischen Datensensoren (3) aus einem Sensor (3) besteht, der es erlaubt, die Anwesenheit und/oder die Konzentration von Hormonen zu bestimmen.

4. Vorrichtung (1) nach einem der vorgehenden Ansprüche, **dadurch gekennzeichnet, dass** einer der besagten physikalischen und/oder physiologischen Datensensoren (3) aus einem Sensor (32) besteht, der es erlaubt, den Widerstand und/oder die Resistivität der vaginalen Schleimhaut und/oder des Schleimes zu bestimmen.

5. Vorrichtung (1) nach einem der vorgehenden Ansprüche, **dadurch gekennzeichnet, dass** einer der besagten physikalischen und/oder physiologischen Datensensoren (3) aus einem oestrogenempfindlichen Sensor (34) besteht.

6. Vorrichtung (1) nach einem der vorgehenden Ansprüche, **dadurch gekennzeichnet, dass** einer der besagten physikalischen und/oder physiologischen Datensensoren (3) aus einer bioaktiven Beschichtung (36) auf dem Gehäuse (2) besteht.

7. Vorrichtung (1) nach einem der vorgehenden Ansprüche, **dadurch gekennzeichnet, dass** einer der besagten physikalischen und/oder physiologischen Datensensoren (3) aus einem Drucksensor besteht.

8. Vorrichtung (1) nach einem der vorgehenden Ansprüche, **dadurch gekennzeichnet, dass** einer der besagten physikalischen und/oder physiologischen Datensensoren (3) aus einem Sensor der Art Kamera besteht.

9. Vorrichtung (1) nach einem der vorgehenden Ansprüche, **dadurch gekennzeichnet, dass** die Mittel zum Verarbeiten (5) der Daten aus wenigstens einem Speicher (51), wenigstens einer Steuereinheit (52) für den Speicher, und wenigstens einer Zentraleinheit (53), die die Verarbeitung der von dem bzw. den physikalischen und/oder physiologischen Datensensoren (3) stammenden Daten sichert, bestehen.

10. Vorrichtung (1) nach Anspruch 9, **dadurch gekennzeichnet, dass** sich die Zentraleinheit (53) im Gehäuse (2) befindet und Speicherkapzität und Verarbeitungssoftware umfasst.

11. Vorrichtung (1) nach einem der vorgehenden Ansprüche, **dadurch gekennzeichnet, dass** die Mittel zum Verarbeiten (5) der Daten eine Signalsender/Empfängereinheit (54) im Gehäuse (2) und eine äussere Zentraleinheit (50) umfasst, wobei die besagten Einheiten (54, 50) geeignet vorgesehen sind, um zu dialogisieren.

12. Vorrichtung (1) nach einem der vorgehenden Ansprüche, **dadurch gekennzeichnet, dass** das Gehäuse (2) außerdem Mittel zum Diffundieren (6) einer Substanz in das Geschlechtssystem des weiblichen Rindes umfasst.

13. Vorrichtung (1) nach Anspruch 12, **dadurch gekennzeichnet, dass** die Mittel zum Diffundieren (6) einer Substanz in das Geschlechtssystem des weiblichen Rindes aus wenigstens einem Elektroventil (60) bestehet, das einem unter Druck stehenden Behälter (63) zugeordnet ist.

14. Vorrichtung (1) nach Anspruch 13, **dadurch gekennzeichnet, dass** das dem unter Druck stehenden Behälter (63) zugeordnete Elektroventil (60) von einer Steuereinheit (62) gesteuert wird.

15. Vorrichtung (1) nach Anspruch 12, **dadurch gekennzeichnet, dass** die Mittel zum Diffundieren (6) einer Substanz in das Geschlechtssystem des weiblichen Rindes aus wenigstens einer am Umkreis des Gehäuses (2) angeordneten diffundierenden und/oder baktericiden Beschichtung (65) besteht.

16. Vorrichtung (1) nach einem der vorgehenden Ansprüche, **dadurch gekennzeichnet, dass** das Gehäuse (2) außerdem einen Ultraschallgenerator umfasst.
